# EUROPEAN PATENT SPECIFICATION

(11) **EP 2 838 915 B1**
(45) Date of publication and mention of the grant of the patent: **30.05.2018**
(21) Application number: 13726299.4
(22) Date of filing: 19.04.2013
(51) Int. Cl.: C07K 14/62

(54) **SOLID PHASE PEPTIDE SYNTHESIS OF INSULIN USING SIDE CHAIN ANCHORED LYSINE**
FESTPHASENPEPTIDSYNTHESE VON INSULIN DURCH SEITENKETTENVERANKERTES LYSIN
SYNTHÈSE PEPTIDIQUE EN PHASE SOLIDE DE L'INSULINE AU MOYEN DE LYSINE FIXÉE À UNE CHAÎNE LATÉRALE

(30) Priority: 20.04.2012 US 201261636193 P
(43) Date of publication of application: 25.02.2015
(73) Proprietor: Chemical & Biopharmaceutical Laboratories Of Patras SA, 26333 Paralia Patras (GR)
(72) Inventor: Barlos, Kleomenis K., Achaea 26000 Patras (GR); Barlos, Konstantinos, 26222 Patras (GR); Gatos, Dimitrios, 26223 Patras (GR); Ziovas, Michail, 26442 Patras (GR); Liopyris, Efstathios, 26333 Patras (GR)
(74) Representative: D Young & Co LLP
(86) International application number: PCT/IB2013/053111
(87) International publication number: WO 2013/156977

(56) References cited:
- WO-A1-2006/082204
- WO-A1-2008/040536
- WO-A2-2004/052916
- WO-A2-2008/109079
- WO-A2-2011/042762
- US-B1- 6 562 942
- BERNHARDT A ET AL: "THE SOLID-PHASE SYNTHESIS OF SIDE-CHAIN-PHOSPHORYLATED PEPTIDE-4-NITROANILIDES", JOURNAL OF PEPTIDE RESEARCH, BLACKWELL PUBLISHING LTD, OXFORD; GB, vol. 50, no. 2, 1 August 1997 (1997-08-01) , pages 143-152, XP000659212, ISSN: 1397-002X
- ALSINA JORDI ET AL: "Solid-phase synthesis of C-terminal modified peptides", BIOPOLYMERS, JOHN WILEY & SONS, INC, US, vol. 71, no. 4, 1 January 2003 (2003-01-01), pages 454-477, XP002468782, ISSN: 0006-3525, DOI: 10.1002/BIP.10492
- MEIENHOFER J ET AL: "SOLID-PHASE SYNTHESIS WITH ATTACHMENT OF PEPTIDE TO RESIN THROUGH AN AMINO ACID SIDE CHAIN: not 8-LYSINE 3/4 -VASOPRESSIN", PROCEEDINGS OF THE NATIONAL ACADEMY OF SCIENCES, NATIONAL ACADEMY OF SCIENCES, US, vol. 68, no. 5, 1 May 1971 (1971-05-01), pages 1006-1009, XP001007913, ISSN: 0027-8424, DOI: 10.1073/PNAS.68.5.1006
- PU WANG ET AL: "Fmoc-Protein Synthesis: Preparation of Peptide Thioesters Using a Side-Chain Anchoring Strategy", INTERNATIONAL JOURNAL OF PEPTIDE RESEARCH AND THERAPEUTICS ; FORMERLY KNOWN AS LETTERS IN PEPTIDE SCIENCE, KLUWER ACADEMIC PUBLISHERS, DO, vol. 11, no. 2, 1 June 2005 (2005-06-01), pages 117-123, XP019287619, ISSN: 1573-3904
- ALSINA J ET AL: "Solid-phase synthesis of ''head-to-tail'' cyclic peptides via lysine side-chain anchoring", TETRAHEDRON LETTERS, PERGAMON, vol. 35, no. 51, 19 December 1994 (1994-12-19), pages 9633-9636, XP026620291, ISSN: 0040-4039, DOI: 10.1016/0040-4039(94)88531-1 [retrieved on 1994-12-19]
- BELGI A ET AL: "The chemical synthesis of insulin: From the past to the present", IMMUNOLOGY, ENDOCRINE AND METABOLIC AGENTS IN MEDICINAL CHEMISTRY 2011 BENTHAM SCIENCE PUBLISHERS B.V. NLD, vol. 11, no. 1, 2011, pages 40-47, XP008164062, ISSN: 1871-5222

## Description

### BACKGROUND OF THE INVENTION

Insulin and insulin derivatives and analogs are prepared efficiently by the solid phase synthesis of the A and B-chains and the random oxidation of the bisoxidized A-chain with linear B-chain. Lysine and lysine containing peptides were attached through the lysine side chain on acid and thermo labile resins. This method allows the solid phase synthesis of various peptides and modified peptides.

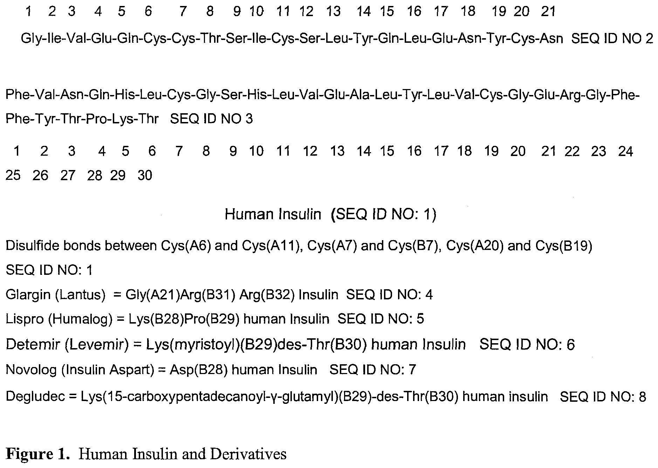

Insulin is a small protein which consists of two peptide chains, the A- and B-chains, which are joined together by two intermolecular disulfide bonds. In addition the A-chain contains an additional intramolecular disulfide bond. Insulin and its derivatives are the most important drugs for the treatment of diabetes. The pharmaceutical properties of insulin can be changed by slight modifications of the two peptide chains. Therefore several insulin derivatives (Figure 1) have been developed and commercialized such as insulin detemir (Levemir), insulin glargin (Lantus), insulin aspart (Novolog), insulin lispro (Humalog) and insulin glulisine (Apidra).

The preparation of insulin using biomimetic approaches are known in the art. P. E. Oyer, S. Cho, J. D. Peterson, D. F. Steiner, J. Biol. Chem. 1971, 246, 1375 - 1386; W. Kemmler, J. D. Peterson, D. F. Steiner, J. Biol. Chem. 1971, 246, 6786 - 6791. These methods produce proinsulin by recombinant DNA techniques, and the linear proinsulin product is then folded by oxidation. Finally, utilizing enzymes, the middle C-peptide of proinsulin is removed and the final insulin is liberated.

To date, a chemical and economically feasible route to insulin has not been developed in spite of numerous efforts from the work of Zahn, Katsogiannis, Kisho, Kent, the Shanghai Institute, Ciba Geigy, Eli Lilly, Novo Nordisc, Sanofi-Aventis and others. None of these efforts have produced synthetic insulin in a reasonable yield and cost.

Four methods which have been applied to date are: 1) the random mixing of the linear A and B chains and their air oxidation; 2) the mixing of A chain containing sulfonic acid groups at the position of the thiol groups of the cysteine residues, with the sulfonated B-chain; 3) the site directed building of the three disulfide bonds between the chains A and B; and 4) a synthesis where the function of the tether C peptide in proinsulin was replaced by an ester bond between the side chains of Glu(A14) and Thr(B30). This method requires two reactions with liquid hydrogen fluoride and nine chromatographic purifications, but this method is considered to be the more effective chemical synthesis to date.

The main difficulties encountered during the chemical synthesis of insulin and its analogues are: a) The insolubility of the A-chain and of intermediate protected peptides which prevent an effective step-by-step solid-phase synthesis and purification; b) The difficult synthesis of the insulin B-chain using Fmoc-amino acids is associated with difficult coupling reaction at positions His(B10), Leu(B11) and Val(B12) [B. Due Larsen and A. Holm J. Peptide Res. 1998, 52, 470-476]; and c) The low yield obtained in the combination of the chains. For example, method 2 (above), gave only a 7% yield of the combined A and B chains.

### SUMMARY OF THE INVENTION:

In one embodiment, the present application discloses effective methods to overcome the above cited problems thus enabling the chemical synthesis of insulin, its derivatives and its analogues. The present application discloses: 1) The synthesis of A chains; 2) B chains; and 3) the combination of the bis-oxidized A and B chains.

In one embodiment, there is provided a method for the solid phase synthesis of a protected, partially protected or unprotected insulin B-chain or an insulin B-chain derivative, the process comprising:
preparing a lysine-resin conjugate comprising a resin and a lysine or a lysine derivative of the formula I, wherein: 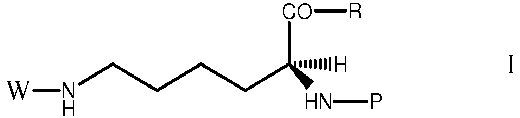 W is a resin of a formulae IIIa, IIIb, IIIc or IIId: 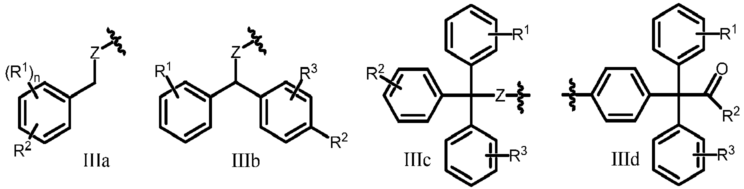 wherein:
n is 0, 1, 2 or 3;
each R¹ and R³ is independently selected from H or is independently selected from the group consisting of 2-Cl, 2-C₁₋₃ alkyl, 2-C₁₋₃ alkoxy, 4-C₁₋₃ alkyl, 4-C₁₋₃ alkoxy, provided that in each of the formulae IIIb, IIIc and IIId, only one of R¹ and R³ is 2-Cl and only one of R¹ and R³ is H;
R² is the solid phase of the resin; and Z is a bond or -C(=O)-;
R is selected from the group consisting of -OH, a carboxyl protecting group, -NH₂, -O-C₁₋₆ alkyl, -O-C₂₋₆ alkenyl, -O-tri-C₁₋₃ alkyl silyl, a peptide residue selected from the group consisting of -Pro-OH, -Pro-NH₂, -Pro-O-C₁₋₆ alkyl, -Pro-O-C₂₋₆ alkenyl, -Pro-O-tri-C₁₋₃ alkylsilyl, Thr(Pr1), -Thr(Pr1)-Arg(Pr2)-Arg(Pr3)-OH, -Thr(Pr1)-Arg(Pr2)-Arg(Pr3)-NH₂, -Thr(Pr1)-Arg(Pr2)-Arg(Pr3)-O-Cl-6 alkyl, -Thr(Pr1)-Arg(Pr2)-Arg(Pr3)-O-C₂₋₆ alkenyl, -Thr(Pr1)-Arg(Pr2)-Arg(Pr3)-O-tri-C₁₋₃ alkylsilyl, -Thr(Pr1)-OH, -Thr(Pr1)-NH₂, -Thr(Pr1)-O-C₁₋₆ alkyl, -Thr(Pr1)-O-C₂₋₆ alkenyl and -Thr(Pr1)-O-tri-C₁₋₃ alkylsilyl, a peptide residue comprising 1 to 200 amino acids comprising optionally protected side chain and optionally protected terminal carboxyl group;
wherein Pr1 is hydrogen or a -OH protecting group and each Pr2 and PR3 is independently hydrogen or a guanidine protecting group;
P is hydrogen, an amino protecting group, an N-terminus peptide residue comprising 1 to 200 amino acids comprising optionally protected side chain and optionally protected terminal amino group, wherein the N-terminus peptide residue comprises a C-terminus and an N-terminus;
provided that when R is selected from the group consisting of -OH, a carboxyl protecting group, -NH₂, -O-C₁₋₆ alkyl, -O-C₂₋₆ alkenyl, -O-tri-C₁₋₃ alkyl silyl, a peptide residue selected from the group consisting of -Pro-OH, -Pro-NH₂, -PrO-O-C₁₋₆ alkyl, -Pro-O-C₂₋₆ alkenyl, -Pro-O-tri-C₁₋₃ alkylsilyl, Thr(Pr1), -Thr(Pr1)-Arg(Pr2)-Arg(Pr3)-OH, -Thr(Pr1)-Arg(Pr2)-Arg(Pr3)-NH₂,-Thr(Pr1)-Arg(Pr2)-Arg(Pr3)-O-C1-6 alkyl, -Thr(Pr1)-Arg(Pr2)-Arg(Pr3)-O-C₂₋₆ alkenyl,-Thr(Pr1)-Arg(Pr2)-Arg(Pr3)-O-tri-C₁₋₃ alkylsilyl, -Thr(Pr1)-OH, -Thr(Pr1)-NH₂, -Thr(Pr1)-O-C₁₋₆ alkyl, -Thr(Pr1)-O-C₂₋₆ alkenyl and -Thr(Pr1)-O-tri-C₁₋₃ alkylsilyl, then P is not hydrogen or an amino protecting group;
coupling the lysine-resin conjugate of the formula I, wherein R is selected from -OH, a carboxyl protecting group and a peptide residue comprising 1 to 200 amino acids comprising optionally protected side chain and optionally protected terminal carboxyl group, with a protected, partially protected or unprotected peptide residue Ib, where the peptide residue comprising 1 to 200 amino acids and the peptide residue Ib together comprises the insulin B-chain or the insulin B-chain derivative, to form the protected, partially protected or unprotected insulin B-chain or an insulin B-chain derivative.

As used herein, a "peptide residue" or "peptide fragment" is a peptide having one or more amino acids. A peptide residue that is attached to the carboxyl terminus, such as that of the lysine-resin conjugate of the formula I, for example, may be a single amino acid that has an alpha-amino protecting group or a carboxyl protecting group, or both protecting groups (i.e., protected or partially protected), or the amino acid lacks an amino protecting group or lacks a carboxyl protecting group (i.e., unprotected or partially protected) or the peptide residue may be dipeptide or polypeptide, wherein each of the amino acids in the peptide may be protected, partially protected or unprotected.
In one aspect of the above, the -OH protecting group Pr1 is an alkyl or benzyl type protecting group such as tert-butyl, 4-methoxy benzyl etc. In another aspect, each Pr2 and Pr3 is independently a guanidine protecting group, a C₁₋₆ alkoxycarbonyl or arylsulfonyl type such as Pbf, Pmc etc.

In one aspect of the above, the amino protecting group of the 1 to 200 amino acids comprising optionally protected side chain, such as Fmoc, is removed by treatment with a secondary amine such as piperidine or diethylamine and the free amine is further coupled with an optionally protected amino acid or an optionally protected peptide comprising 1 to 200 amino acids using a coupling agent such as DCC, DIC or EDAC, optionally in the presence of an additive selected from HOBt, HOSu, thiophenol or pentafluorophenol.

As used in reference to the compound of the formula I, the clause "P is an N-terminus peptide residue comprising 1-200 amino acids" means that the 1-200 amino acids is attached to the N-terminus (or the alpha-amino group) of the lysine or lysine derivative. In one aspect, the activation of the C-terminus free carboxyl group is performed in a solvent selected from the group consisting of DCM, DMF, NMP, DMSO or mixtures thereof.

In one aspect of the above method, the resin is a 2-chlorotrityl resin. In another aspect of the above, P is selected from the group consisting of tert-butyloxycarbonyl (Boc), 9-fluorenylmethyloxycarbonyl (Fmoc), benzyloxy-carbonyl (carboxybenzyl or Z), 1-(4,4-dimethyl-2,6-dioxocyclohex-1-ylidene)-ethyl (Dde), 2-nitrophenylsulfenyl (Nps) and allyloxycarbonyl (alloc). In another aspect of the method, the partially protected or unprotected insulin B-chain or an insulin B-chain derivative is selected from the group consisting of SEQ ID NOs: 4, 5, 6, 7 and 8, and analogs or derivatives thereof; and further cleaving of the resin-bound insulin B-chain or an insulin B-chain derivative by contacting the resin-bound peptide under mild acidic condition using a mixture of an organic acid and an alcoholic solvent, or by heating the resin-bound peptide to an elevated temperature, or both using a mixture of an organic acid and an alcoholic solvent along with heating the resin-bound peptide at an elevated temperature for a sufficient period of time to cleave the insulin B-chain or an insulin B-chain derivative from the resin. In another aspect of the method, the organic acid is selected from the group consisting of trifluoroacetic acid and acetic acid, and mixtures thereof, the alcoholic solvent is selected from the group consisting of trifluoroethanol, hexafluoro-isopropanol, methanol and mixtures thereof, and heating of the resin bound peptide is performed with microwaves. In another aspect of the method, the partially protected or unprotected insulin B-chain or an insulin B-chain derivative is selected from the group consisting of an N-acylated derivative, a pegylated derivative, a biotinylated derivative, a derivative comprising a chromophore and a peptide residue comprising a natural amino acid residue, an unnatural amino acid residue, and mixtures thereof.

In another embodiment, there is provided a method for preparing an insulin B-chain peptide selected from SEQ ID NOs: 3, 23, 27, 29 and 34 comprising cleaving the peptide from a peptide-resin conjugate of the formula IV:

W-AA₁-AAₘ IV

wherein: W is a resin of a formulae IIIa, IIIb, IIIc or IIId wherein n, R¹ R² and R³ and Z are as defined above, wherein:
AA₁ is a first peptide residue comprising a lysine residue or derivative thereof attached to W by the amino side chain of the lysine or lysine derivative;
and AAₘ is the second to m number of residues where m is an integer from 1-200; by contacting the peptide-resin conjugate with a mixture of an organic acid and a solvent, or by heating the peptide-resin to an elevated temperature, or both using a mixture of an organic acid and a solvent, along with heating the resin-bound peptide at an elevated temperature for a sufficient period of time to cleave the peptide residue from the resin W. In one aspect of the method, the peptide-resin conjugate is selected from the group consisting of SEQ ID NOs: 17, 22 and 24. In another aspect of the method, the peptide-resin conjugate is selected from SEQ ID NO. 17 is further deprotected to form human B-chain of SEQ ID NO: 3. In another aspect of the above method, the organic acid is selected from the group consisting of trifluoroacetic acid and acetic acid, and mixtures thereof, the alcoholic solvent is selected from the group consisting of trifluoroethanol, hexafluoro-isopropanol, methanol and mixtures thereof, and heating of the peptide-resin conjugate is performed at about 30 to 50 °C. In another aspect of the above method, the cleavage of the resin-bound peptide is performed by heating the resin-bound peptide at an elevated temperature in an alcoholic solvent with the intermittent addition of about 0.01 wt/wt%, 0.02 wt/wt%, 0.03 wt/wt%, 0.05 wt/wt%, 0.1 wt/wt%, 0.2 wt/wt%, 0.5 wt/wt%, 1.0 wt/wt%, 2.0 wt/wt%, 3.0 wt/wt%, 4.0 wt/wt% or about 5.0 wt/wt% of the organic acid. The acid may be added at intervals of about 5 minutes, 10 minutes, 15 minutes, 30 minutes or about 1 hour. In another aspect of the method, heating the resin-bound peptide is performed at an elevated temperature of about 25 - 30 °C, 30 - 35 °C or 35 - 40 °C. In another aspect of the method, the cleavage may be enhanced with an additive that accelerates coupling reactions in peptide synthesis selected from hydroxybenzotriazol, hydroxysuccinimide, pentafluorophenol and thiophenol. In another aspect, the partially protected or unprotected insulin B-chain or an insulin B-chain derivative is selected from the group consisting of an N-acylated derivative, a pegylated derivative, a biotinylated derivative, a derivative comprising a chromophore and a peptide residue comprising a natural amino acid residue, an unnatural amino acid residue, and mixtures thereof. In another aspect of the above, the derivative is a variant of the insulin B-chain or insulin B-chain derivative.

Generally, when the peptide is cleaved from the resin, further peptide synthesis may be performed by solution peptide synthesis, solid phase peptide synthesis or a combination of solution and solid phase peptide synthesis, also referred to as phase change synthesis. In addition, because a lysine has an amine side chain, a alpha-amino group and a carboxyl group, peptide coupling reactions with a lysine, that may be attached to the resin by the side chain or by the carboxyl group, there are multiple permutations for peptide synthesis (solid phase or solution phase) to prepare linear and/or branched peptides. That is, when the lysine containing peptide is cleaved from the resin, further solution phase peptide synthesis may include various combinations of the steps of: 1) Protecting the free amine group of the lysine side chain; 2) deprotecting the C-terminal carboxyl group and coupling of the free carboxyl group with an amino acid or peptide fragment, or derivatives thereof; 3) deprotecting the C-terminal carboxyl group and attaching the free carboxyl group to the resin and further performing solid phase peptide synthesis by peptide coupling to the N-terminal or peptide coupling to the amine side chain of the lysine (to prepare branched peptides); 4) deprotecting the N-terminal amino group (alpha-amino group) and coupling of the free amine with an amino acid or peptide fragment; and 5) combinations thereof.

### DEFINITIONS:

As used herein, an "acid sensitive" resin, a "thermal sensitive" resin or both an "acid and thermal sensitive" resin is a resin, such as the resin in the lysine resin-conjugate, that detaches from the lysine or lysine derivative under mild acidic conditions, under relatively low temperatures or both under mild acidic conditions and relatively low temperatures, and the resin detaches under such conditions that does not result in the undesired side reactions, undesired deprotection of a selected protecting group, global deprotection or racemization of the lysine, lysine derivatives or peptides.

As used herein, a "branched peptide" is a peptide that may be prepared according to the methods described herein, wherein amino acids or peptide residues may be attached to 1) the amino-side chain of a lysine group, 2) the C-terminal carboxyl group, and 3) the N-terminal amino group.

As used herein, "global deprotection" means that under certain deprotecting conditions, such as a strongly acidic acid condition, the condition result in the deprotection of all protecting groups of the lysine derivative or peptide on the resin. In one aspect, the peptide residue comprising 1 to 200 optionally protected amino acids comprise an amino protecting group P on the amino acid, wherein each P on the amino acid residue is hydrogen (i.e., unprotected) or is independently selected from the group consisting of tert-butyloxycarbonyl (Boc), 9-fluorenylmethyloxycarbonyl (Fmoc), benzyloxy-carbonyl (carboxybenzyl or Z), 1-(4,4-dimethyl-2,6-dioxocyclohex-1-ylidene)-ethyl (Dde), 2-nitrophenylsulfenyl (Nps) and allyloxycarbonyl (alloc).

As used herein, "optionally protected" means that the peptide residue comprising a single amino acid or a peptide residue, and wherein each amino group and each carboxyl group of the peptide residue may be independently protected or unprotected. In one aspect, the peptide residue or peptide fragment contains at least 2 amino acid residues, at least 10 amino acid residues or at least 200 amino acid residues.

As used herein, a "carboxyl protecting group" for example, as represented in the compound of the formula I, means that the R group that is attached to -C(O)- group includes the oxygen of the -C(O)- group and further comprises the protecting group as known in the art and as described herein. A comprehensive list of suitable protecting groups may be found in T. W. Greene, Protecting Groups in Organic Synthesis, 3rd edition, John Wiley & Sons, Inc. 1999.

As used herein, a "variant" means a peptide substantially homologous to a native peptide or derivative, but which has one or more amino acid sequence that is different from the native peptide or peptide derivative that are based one or more deletions, insertions or substitutions. Amino acid sequence insertions include amino- and/or carboxyl-terminal fusions ranging in length from one residue to peptides containing a ten, twenty or thirty or more residues, as well as intrasequence insertions of single or multiple amino acid residues. Intrasequence insertions, that is, insertions within the desired polypeptide sequence, may range generally from about 1 to 10 residues, 1 to 5 or 1 to 3 residues. Variants can comprise conservatively substituted sequences, meaning that a given amino acid residue is replaced by a residue having similar physiochemical characteristics. See, Zubay, Biochemistry, Addison-Wesley Pub. Co., (1983). It is a well-established that certain amino acids substitutions, i.e., "conservative" amino acid substitutions, can frequently be made in a protein or a peptide without altering either the confirmation or the function of the protein or peptide. Such changes include substituting any of isoleucine (I), valine (V), and leucine (L) for any other of these amino acids; aspartic acid (D) for glutamic acid (E) and vice versa; glutamine (Q) for asparagine (N) and vice versa; and serine (S) for threonine (T) and vice versa. Variants will have an amino acid sequence having at least 90% amino acid sequence identity with the reference sequence, at least 95%, at least 97%, at least 98% or at least 99% amino acid sequence identity. The variants will retain the primary function of the parent from which it they are derived.

### Brief Description of the Figures:

Figure 1 shows the sequence of human insulin and various derivatives
Figure 2 is a representative process scheme for a solid phase synthesis of bisoxidized human insulin A-chain isomers.
Figure 3 is a representative scheme showing a step-by-step solid-phase synthesis of the insulin B-chain using a CTC-resin.
Figure 4 is a representative scheme showing the synthesis of an insulin B-Chain using Fmoc-Lys-Thr(tBu)-OH attached through the side chain of Lys on an MMT-resin.
Figure 5 is a representative scheme for the solid-phase synthesis of Fmoc-Lys-Thr(tBu)-Arg(Pbf)-Arg(Pbf)-OH (SEQ ID NO: 19) for Glargin B-Chain.
Figure 6 is a representative scheme for the synthesis of insulin Glargin B-Chain using Fmoc-Lys-Thr(tBu)-Arg(Pbf)-Arg(Pbf)-OH attached through the side chain of Lys on a MMT-resin.
Figure 7 is a representative scheme for the synthesis of insulin Detemir B-chain with N-terminal protection and using Fmoc- Lys attached through the side chain onto a MMT-resin.
Figure 8 is a representative scheme for the synthesis of insulin Degludec B-Chain using Fmoc- Lys attached through the side chain on MMT-resin.
Figure 9 is a representative scheme for the synthesis of insulin Degludec B-chain using N-terminal protection.

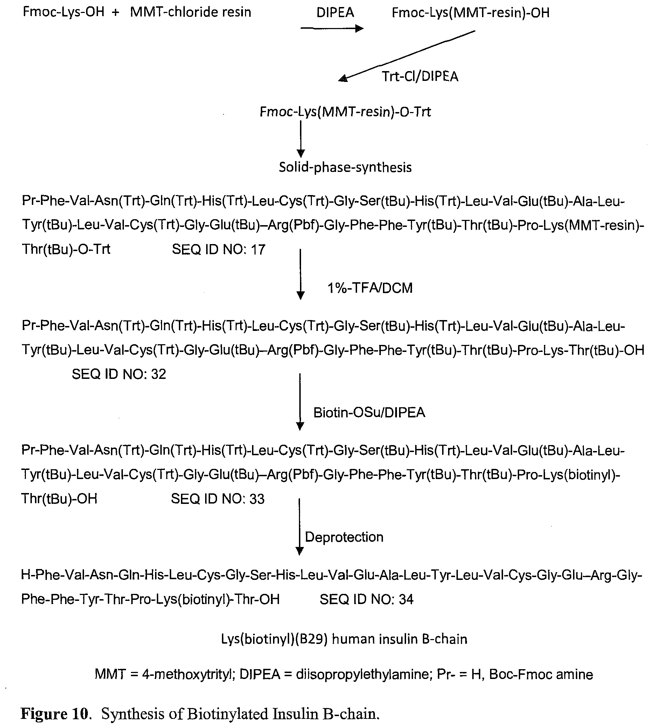

Figure 10 is a representative scheme for the synthesis of biotinylated insulin B-chain.
Figure 11 is a representative scheme for the synthesis of insulin and insulin analogs (Lispro and Aspart) by chain combination.
Figure 12 is a representative scheme for the chain combination of an insulin analogs (Detemir and Degludec).
Figure 13 is a representative scheme for the chain combination of an insulin analog Glargin.

### Synthesis of A Chains:

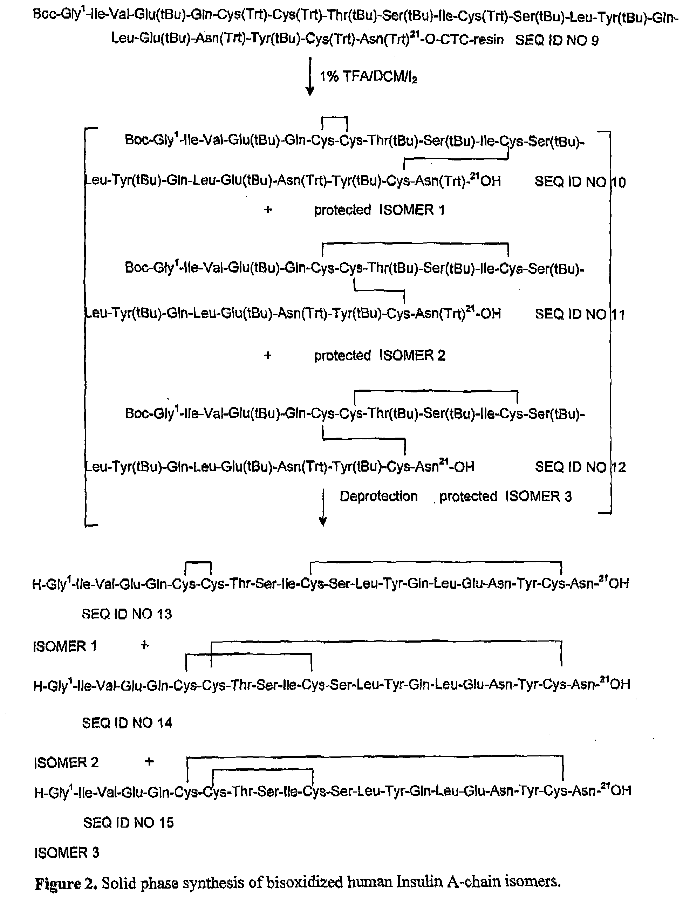

In one embodiment, there is provided a method for the solid-phase synthesis of the insulin bis-oxidized A-chain in which the cleavage of the protected peptide from the resin and its oxidation proceeds concurrently and in a short period of time. In this method, the synthetic problem due to the insolubility of the A-chain is overcome. In one aspect, this is achieved by applying solid-phase-synthesis using acid labile resins. Such resins include the trityl, diphenylmethyl and benzyl-type. The peptide chain is assembled using standard protocols for the solid phase synthesis of peptides using, for example, Fmoc-amino acids (Figure 2). Oxidation of the resin-bound peptide maybe performed using iodine under mild acidic conditions, such as in halogenated hydrocarbons. Under these conditions, the cleavage of the protected peptide from the resin proceeds concurrently with the oxidation reaction, and the precipitation of the A-chain is avoided. In one aspect, solutions of trifluoroacetic acid or acetic acid in dichloromethane that are mixed with alcohols, such as trifluoroethanol or methanol, were used as the solvent for the concurrent oxidation and cleavage from the resin. In one embodiment, 2-chlorotrityl resin was used for the solid-phase chain assembly of the protected A-chain. The bis-oxidized protected linear A-chain that was obtained was deprotected by contacting the A-chain with various acidic solvent solutions, including DCM, TFA, TES and thioethers, and mixtures thereof. In one aspect, the present application discloses all three expected isomers of the bis-oxidized insulin A-chain (Figure 2). The isomers can be separated by HPLC but can be also isolated and purified as a mixture of isomers.

### Synthesis of B Chains

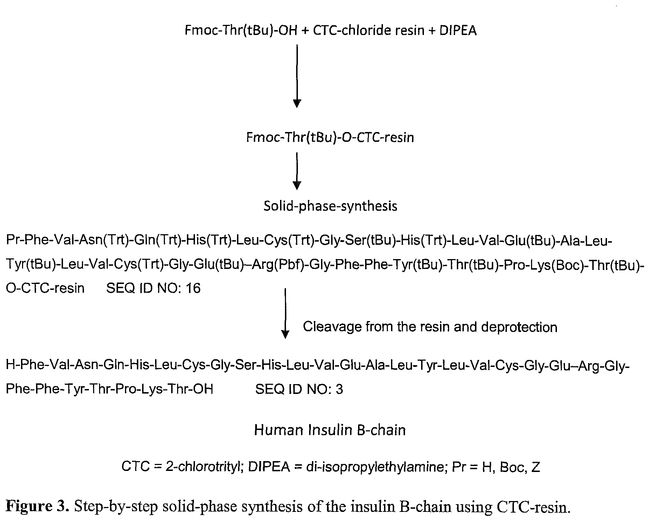

In one embodiment, the present application discloses a synthesis of the insulin B-chain in a step-by-step manner on acid sensitive resins of the trityl-type, such as the 2-chlorotrityl resin (Figure 3). The application of Fmoc-amino acids suitably protected at their side chains with acid sensitive groups was also employed.

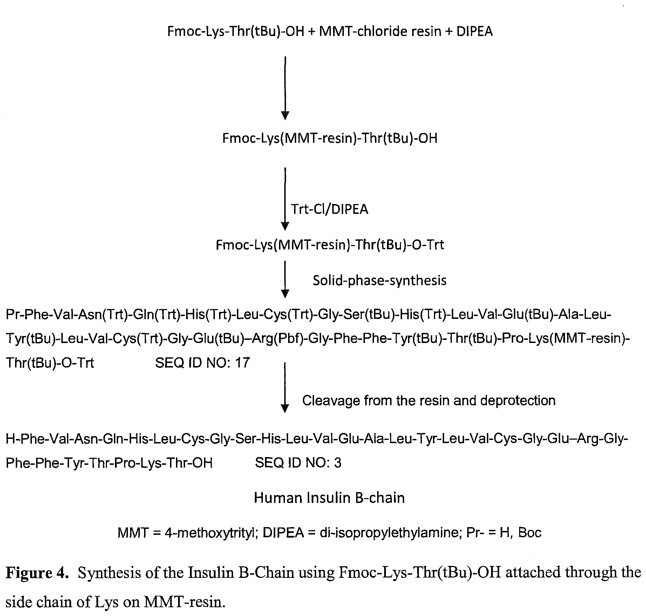

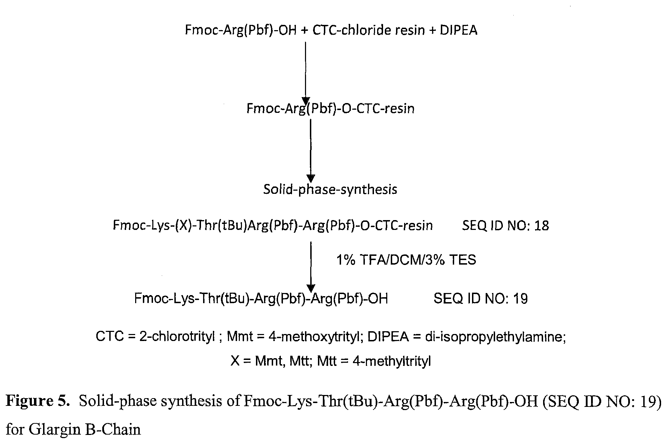

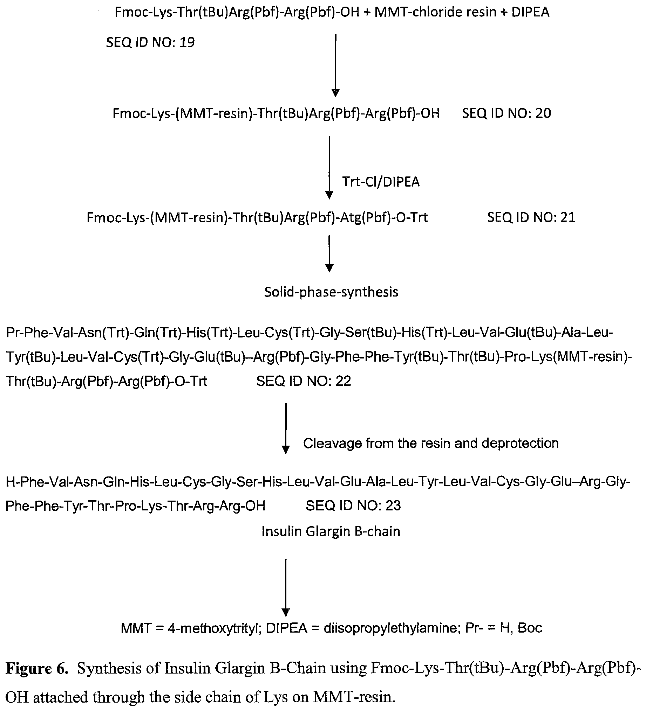

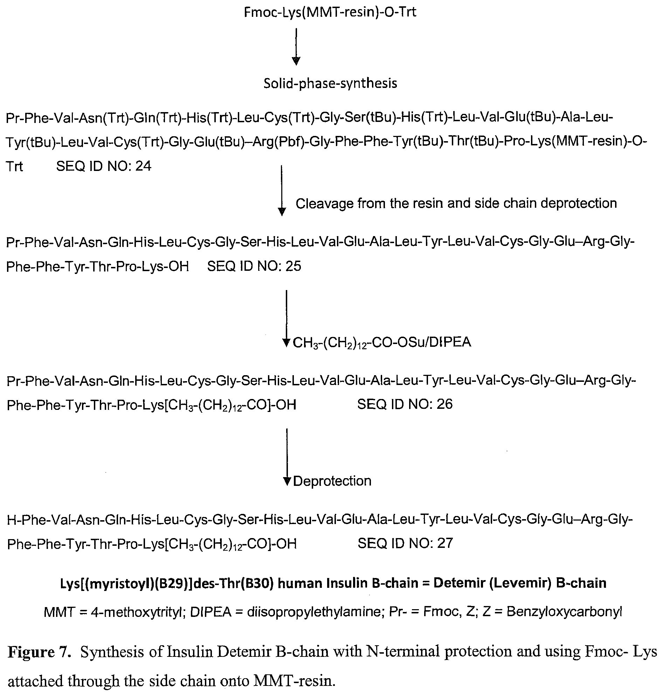

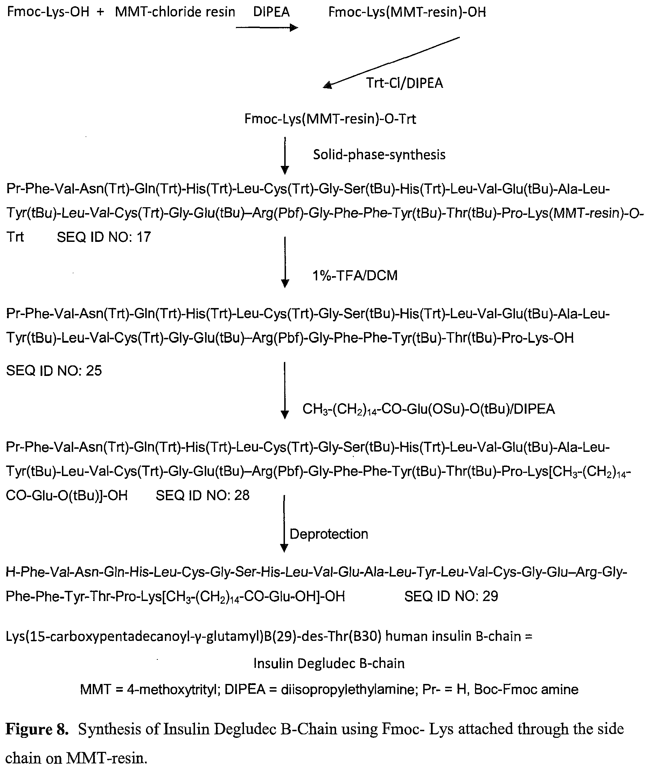

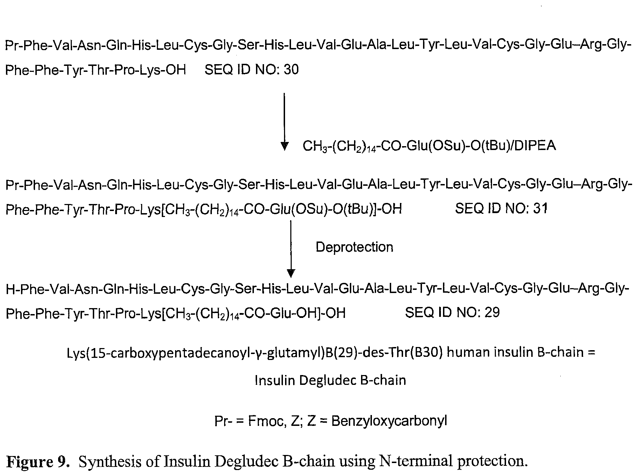

The present application also discloses the synthesis of insulin B-chain peptides and its derivatives such as the des-Thr(B30) insulin B-chain may be prepared where the side chain of Lys(B29) is attached to the resin instead of the chain's carboxyl group (Figures 4). Similarly the B-chain for Glargin may be synthesized either by solid phase attachment of the protected arginine through the carboxyl group (Figure 5) or using side group attachment through the lysine (Figure 6). Furthermore, the side chain of the lysine that is attached to the resin may be used to prepare the Insulin Detemir B-chain (Figure 7), and Insulin Degludec B Chain (Figure 8, 9).

The present application further discloses that if the resin used for the side chain attachment of Lys is relatively labile, such as the 2-chlorotrityl resin or the 4-methoxytrityl resin, the partially protected insulin B-chain can be readily obtained by mild acidic or thermal treatment of the resin-bound peptide, where the peptide is cleaved from the resin with selectively deprotected Lys side-chain amino function. The partially deprotected insulin B-chains, their shorter or longer fragments and derivatives, can be selectively acylated in solution at the lysine side chain providing a variety of important B-chain derivatives.

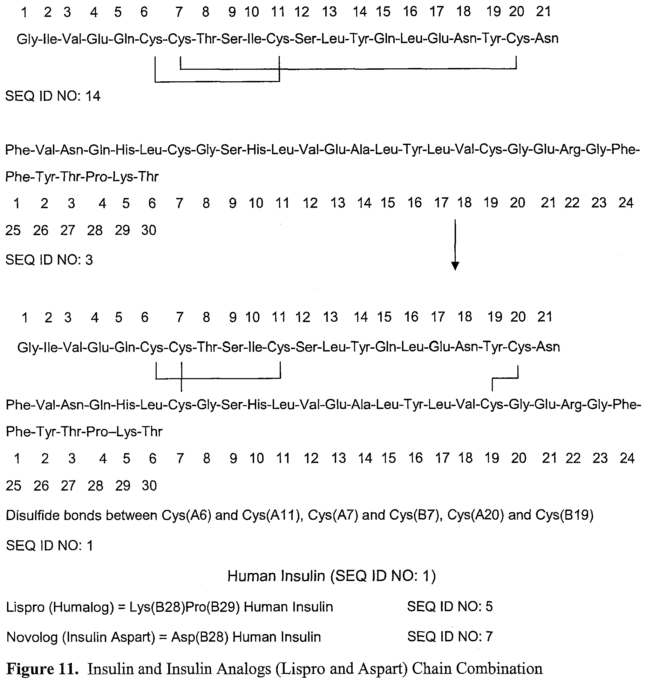

The present application further discloses the synthesis of Lys(15-myristoyl)-des-Thr(30) human insulin B-chain, the synthesis of Lys(15-carboxypentadecanoyl-γ-glutamyl)B(29)-des-Thr(B30) human insulin B-chain and the selective pegylation and biotinylation (Figure 11) of the side chain of Lys(B29) as well as the solid-phase-synthesis of selectively at the Lys(B29) side chain branched Insulin B-chain peptides.

The present application also discloses the selective acylation of the side chain of the Lys(B29)-human insulin B-chain and its shorter and longer peptide analogues and their derivatives. This can be performed by preparing and isolating the insulin B-chain protected at its amino terminal function by the Fmoc-group or a Z-type group. After the removal of the side chain protecting groups of the insulin B-chain derivative, the free amino function of the Lys(B30)-insulin may be acylated.

### Combining Bisoxidized Chain A with B

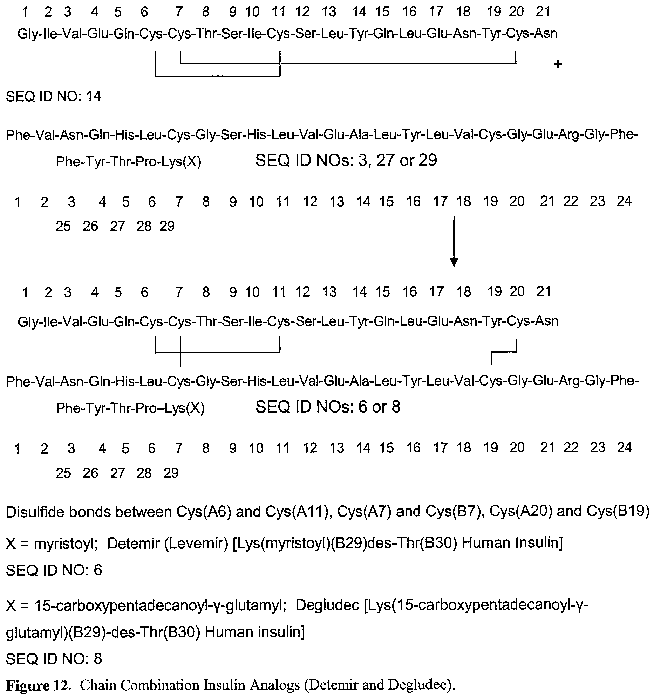

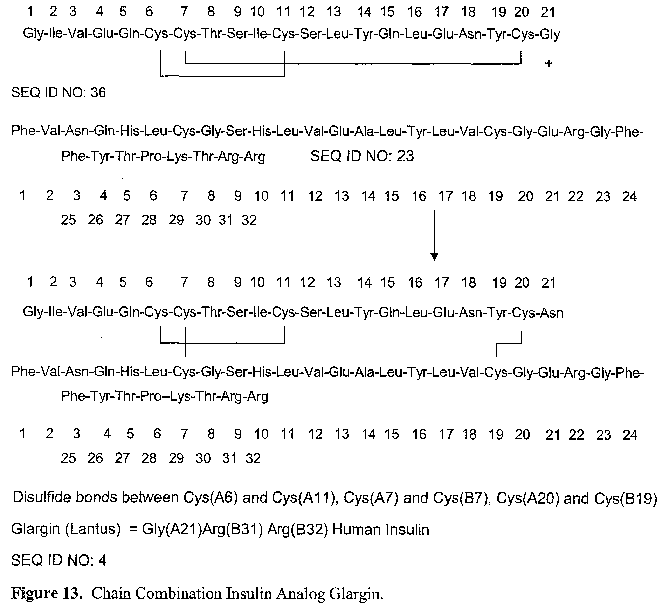

The present appliction further discloses that the combination of the insulin chains using the bisoxidized A-chain of human or animal insulin and their derivatives, with the B-chain of human or animal insulin and their derivatives proceed smoothly in aqueous solutions buffered with the addition of various salts, such as sodium, calcium, zinc, iron salts etc. Furthermore, the solution may contain organic solvents such as alcohols, DMSO, acetonitrile etc. at various pH, including at pH > 7, and at different temperatures, including from about 0-5 °C, 2-6 °C and 5-10 °C. The bis-oxidized A-chain and the B-chain can be reacted at different ratios, such as a ratio where the A-chain/B-chain ratio is >1, such as 1.05:1, 1.1:1, 1.2:1, 1.3:1, 1.5:1 and 2:1. In one aspect, the reaction provides a mixture of mono-oxidized A-chain, oxidized B-chain and B-chain dimers, A-chain dimmers and mixtures thereof. The product mixture may be separated by HPLC and recycled after the oxidation of the mixture of mono and di-oxidized A-chain, or converted to different insulin products by equilibrating with a redox system such as cysteine cysteine, or oxidized and reduced glutathione etc. Using the present method, combination yields of > 60% may be obtained. Alternatively, a mild oxidant is added to the combination mixture, such as DMSO or the redox mixture oxidized and reduced glutathione, to re-oxidize the A-chain. In the resulting mixture, a mild reducing agent, such as thiols, including thiolamine, dithiotreitol or a redox system, may be added. Using the present method, the total yield of insulin and insulin analogs may be improved by 5-25% (Figures 11-13).

The preparation of insulin, insulin analogues and acylated insulin analogues are described in the examples that follows. The reaction and purification schemes described are generally applicable to the preparation of various different insulin derivatives, but the reactions conditions and sequences may not be applicable to all peptides, including certain insulin analogues and derivatives, as would be readily recognised by those skilled in the art. In these cases, the reactions can be successfully performed by usual modifications known to those skilled in the art in peptide synthesis, that is, by appropriate protection of interfering groups, changing to other conventional reagents or routine modification of reaction conditions and reaction sequences.

### EXAMPLES

### Example 1

Solid-phase synthesis of insulin A chain, B chain and of their protected segments General procedure:

### A1. Preparation of loaded 2-chlorotrityl resins

2-Chlorotrityl chloride resin (CTC-C1) (100 g; loading 1.6 mmol/g) of CBL-Patras, was placed in a 2 L peptide synthesis reactor and swelled with 700 mL dichloromethane (DCM) for 30 min at 25 °C. The resin was filtered and a solution of 100 mmol Fmoc-amino acid and 300 mmol diisopropylethylamine (DIEA) in 500 mL DCM was added. The mixture was stirred under nitrogen for 2 hours at 25 °C. The remaining active sites of 2-CTC resin were neutralised by adding 10 mL of methanol (MeOH) and reacting for 1 hour. The resin was filtered and washed twice with 400 mL DMF. The resin was filtered and treated twice with 500 mL 25% by volume of piperidine in DMF for 30 min. The resin was washed four times with 500 mL DMF. The resin was unswelled with 3 washes with 500 mL of isopropanol (IPA); and dried to constant weight. 70-95% of the mmol of the used amino acid was bound on the resin.

### A2. Preparation of loaded MBH-resins, a general method

MBH-Br resin (100 g; 190 mmol) was placed in a 2 L peptide synthesizer and swollen with 700 mL DCM for 30 min at 25 °C. The resin was filtered and then a solution of Fmoc-amino acid and DIEA in 500 mL DCM was added. The mixture was stirred under nitrogen for 6 h at 25 °C. Then the remaining active sites of the MBH resin were bound by adding 10 mL MeOH and stirring for 24 h. The resin was then filtered and washed twice with 400 mL DMF. The resin was filtered and reacted twice with 500 mL of a solution of 25% by volume of piperidine in DMF for 30 min. The resin was then washed four times with 500 mL DMF. The resin was diswelled with three washes with 500 mL IPA. The resin was then dried to constant weight under vacuum (15 torr, 25 °C). 60-90% of the mmol of the used amino acid were bound onto the resin.

### B. Solid-phase synthesis, a general protocol

The solid-phase synthesis was performed at 24 °C, with 1.0 g amino acid esterified to the CTC or MBH resin as described in Part A of Example 1. During the whole synthesis the following protocol was used.

### B1. Swelling of the resin

The resin was placed in a 15 ml reactor and treated twice with 7 mL NMP, followed by filtration.

### B2. Activation of the amino acid

The amino acid (3.0 equiv.) and 1-hydroxybenzotriazol (4.0 equiv.) was dissolved in a reactor with 2.5 times their volume in NMP and cooled to 0 °C. DIC was then added (3.0 equiv.) and the mixture was stirred for 15 min.

### B3. Coupling Reaction

The solution which was prepared in B2 was then added to the B1 reactor. The reactor was washed once with one volume of DCM and was added to the reactor which was stirred for 1-3 h at 25°-30 °C. A Kaiser Test was performed to determine the completion of the reaction. If the coupling reaction was not completed after 3 h (positive Kaiser Test), the reaction mixture was filtered and recoupled with a fresh solution of activated amino acid. After completion of the coupling the reaction mixture was filtered and washed 4 times with NMP (5 volumes per wash).

### B4. Removal of the Fmoc-group

The resulting resin in B3 was filtered and then treated for 30 min with 5 mL of a solution which contained 25% by volume of piperidine. The resin is washed 3 X 5 mL NMP.

### B5. Elongation of the peptide chain

After the incorporation of each amino acid the steps B1-B5 were repeated until the desired peptide chain was formed.

The following Fmoc-amino acids were used for coupling of the individual amino acid or amino acid fragments: Fmoc-Gly-OH, Fmoc-Ala-OH, Fmoc-Val-OH, Fmoc-Ile-OH, Fmoc-Leu-OH, Fmoc-Phe-OH, Fmoc-Pro-OH, Fmoc-Asp(tBu)-OH, Fmoc-Glu(tBu)-OH, Fmoc-Lys(Boc)-OH, Fmoc-Lys(Mmt)-OH, Fmoc-Lys(Mtt)-OH, Fmoc-Ser(tBu)-OH, Fmoc-Ser(Trt)-OH, Fmoc-Thr(tBu)-OH, Fmoc-Thr(Trt)-OH, Fmoc-Tyr(tBu)-OH, Fmoc-Tyr(Clt)-OH, Fmoc-Asn-OH, Fmoc-Asn(Trt)-OH, Fmoc-Gln-OH, Fmoc-Gln(Trt)-OH, Fmoc-Arg(Pbf)-OH, Fmoc-His(Trt)-OH, Fmoc-Cys(Trt)-OH, Fmoc-Cys(Mmt)-OH and Fmoc-Cys(Acm)-OH; and the following Boc-amino acids: Boc-Phe-OH, and Boc-Gly-OH.

C. General method for the cleavage from the CTC- resin of the partially protected insulin peptides and of their protected fragments which contain Fmoc- or Boc-groups on their N-terminus and are selectively deprotected at a lysine side chain.

The resin-bound peptide or peptide segment which was produced as described above in B1-B5 and was protected at a specific Lys side chain with Mmt or Mtt, was washed 4 times with 5 mL NMP, 3 times with 5 ml IPA and finally 5 times with 7 ml DCM to remove completely any residual NMP or other basic components. The resin was then cooled to 0 °C, filtered from DCM and was treated six times with a solution of 10 mL 1.0-1.5% TFA in DCM/TES(95:5) at 5 °C. The mixture was then stirred 20 min at 0 °C and filtered. The resin is then washed three times with 10 mL DCM. Pyridine is then added to the filtrates (1.3 equiv. relative to TFA) to neutralize the TFA. The cleavage solution in DCM was then mixed with an equal volume of water. The resulting mixture was distilled at reduced pressure to remove DCM (350 torr at 28 °C). The peptide or peptide fragment precipitated after the removal of DCM. The resulting peptide was washed with water and ether and dried at 30-35 °C under 15 Torr vacuum. Alternatively DCM was removed in vacuum and the partially protected peptide was precipitate by the addition of ether.

D. Cleavage from the CTC-resin and simultaneous mono-oxidation of protected peptides with iodine. Preparation of bis-oxidized Insulin A-chains. General procedure.

The resin bound on the N- and on the side chains protected peptide, obtained as described above, was washed 4 X 5 mL NMP, 3 X 5 ml IPA and finally 5 times with 7 ml DCM to remove completely NMP and other basic components. The resin was then cooled to 0 °C. After filtration of DCM the resin was processed twice at 5 °C with a solution of 10 mL 1%-TFA in DCM containing 20 equivalents (equiv.) of iodine in relation to the on the resin bound peptide. The resulting mixture was stirred for 5 min at 0 °C and filtered (instead of 1% TFA the same volume of a mixture of dichloromethane/acetic acid/trifluoroethanol can be used with similar results). The resin was then washed three times with 10 mL DCM. The combined filtrates were heated to 15 °C and stirred for further 30 min. Pyridine was then added to the filtrates (1.3 equiv. relative to TFA) to neutralize TFA. The cleavage solution in DCM was then mixed with an equal volume of 3%-sodium thiosulphate in water in order to remove the excess iodine. This was indicated by the discoloration of the mixture. The resulting mixture was distilled at low pressure to remove DCM (350 torr at 28 °C). The resulting peptide or peptide fragment precipitated out after the removal of DCM. The resulting peptide was washed with water and dried at 30-35 °C under vacuum of 15 Torr.

### Example 2

### Deprotection of the bis-oxidized insulin A-chains described in Figure 2. General method:

The protected insulin chain A obtained as described above in Example 1 (0.01 mmol) were treated with 10 mL TFA/TES/thioanisol/water (85:5:5:5) for 3 h at 5 °C and for 1 h at 15 °C. The resulting solution was concentrated in vacuum and then the deprotected peptide was precipitated by the addition of diisopropylether and washed three times with 10 mL diisopropylether. The resulting solid was dried in vacuum (25 °C, 15 Torr) until constant weight.

### Example 3

### Deprotection of the bisoxidized insulin B-chains. General method:

The protected insulin chain B obtained as described above in Example 1 (0.01 mmol) was treated with 10 mL TFA/DTT/water (90:5:5) for 3 h at 5 °C and for 1 h at 15 °C. The resulting solution is concentrated in vacuum and then the deprotected peptide was precipitated by the addition of diisopropylether and washed with 3 X 10 mL diisopropylether. The resulting solid was dried in vacuum (25 °C, 15 Torr) until constant weight.

### Example 4

### Synthesis of peptides attached on resins through the side chain of Lysine.

1 mmol of a Lys side chain deprotected amino acid or peptide, which can be obtained as described in the example 1C, was dissolved in 15 ml DCM. Then, 1.5 mmol DIPEA was added and 1 g 4-methoxytrityl chloride resin (1.2 mmol/g) and the mixture was stirred over night. 1 ml methanol was added and the mixture was stirred for additional 4 h at RT. The resin was then filtered, washed 3 X DCM, 3 X DMF, 3 X iPrOH and 3 X hexanes and dried in vacuum to constant weight.

### Example 5

### Synthesis of selectively at the Lysine side chain acylated peptides. General procedure.

1 mmol of a Lys side chain deprotected amino acid or peptide, which can be obtained as described in the example 1C, was dissolved in 15 ml DMF. Then, 1.2 mmol DIPEA were added and 1 equivalent of the electrophilically activated agent and the mixture was stirred for 1-12 h at RT. The mixture was then poured into ice cold water and the resulting precipitate was washed with water and ether, deprotected as described under example 3 and purified as described under example 6 below.

### Example 6

### Purification of the deprotected peptides. General procedure.

Crude deprotected trifluoroacetic acid salts of the insulin chains and of the bicyclic chain A derivatives were dissolved in 15% acetonitrile in water and loaded on a semi-preparative column 10x25 mm loaded with Chromasil; Phase A = 1%-TFA in acetonitrile, phase B = 1%-TFA in water; Linear gradient from 25%-A to 65%-A in 30 min. The purification yield varied from 30 to 90%.

### Example 7

### Synthesis of insulin like peptides and of their derivatives by the linear combination of the bicyclic A-chain of the insulin and of its derivatives and of the linear B-chain of insulin and of its derivatives: General procedure.

Deprotected bicyclic A-chain of insulin like peptide or of its derivatives (0.006 mmol) and of linear chain-B of insulin like peptides or derivatives (0.005 mmol) was dissolved in 4 ml of a buffer of sodium glycinate/6-N guanidine hydrochloride (4:1) at pH = 10.5. Then 1 ml DMSO was added gradually within 12 hours and then the mixture was stirred for additional 4 h at 15 °C. From the resulting solution, the insulin-like peptides were isolated by purification performed as described in Example 4. The average yield of three experiments on insulin-like peptides was 5-80% calculated on the applied B-chain.

While a number of exemplary embodiments, aspects and variations have been provided herein, those of skill in the art will recognize certain modifications, permutations, additions and combinations and certain sub-combinations of the embodiments, aspects and variations. It is intended that the following claims are interpreted to include all such modifications, permutations, additions and combinations and certain sub-combinations of the embodiments, aspects and variations are within their scope. The entire disclosures of all documents cited throughout this application are incorporated herein by reference.

## Claims

1. A method for the solid phase synthesis of a protected, partially protected or unprotected insulin B-chain or an insulin B-chain derivative, the process comprising:
preparing a lysine-resin conjugate comprising a resin and a lysine or a lysine derivative of the formula I, wherein: 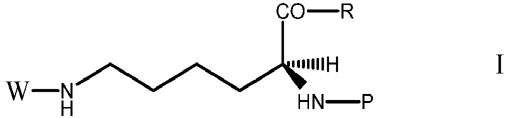 W is a resin of a formulae IIIa, IIIb, IIIc or IIId: 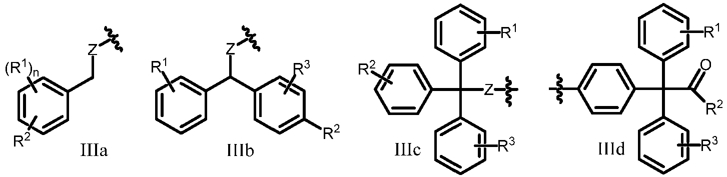 wherein:
n is 0, 1, 2 or 3;
each R¹ and R³ is independently selected from H or is independently selected from the group consisting of 2-Cl, 2-C₁₋₃ alkyl, 2-C₁₋₃ alkoxy, 4-C₁₋₃ alkyl, 4-C₁₋₃ alkoxy, provided that in each of the formulae IIIb, IIIc and IIId, only one of R¹ and R³ is 2-Cl and only one of R¹ and R³ is H;
R² is the solid phase of the resin; and Z is a bond or -C(=O)-;
R is selected from the group consisting of -OH, a carboxyl protecting group, -NH₂, -O-C₁₋₆ alkyl, -O-C₂₋₆ alkenyl, -O-tri-C₁₋₃ alkyl silyl, a peptide residue selected from the group consisting of -Pro-OH, -Pro-NH₂, -Pro-O-C₁₋₆ alkyl, -Pro-O-C₂₋₆ alkenyl, -Pro-O-tri-C₁₋₃ alkylsilyl, Thr(Pr1), -Thr(Pr1)-Arg(Pr2)-Arg(Pr3)-OH, -Thr(Pr1)-Arg(Pr2)-Arg(Pr3)-NH₂, -Thr(Pr1)-Arg(Pr2)-Arg(Pr3)-O-C1-6 alkyl, -Thr(Pr1)-Arg(Pr2)-Arg(Pr3)-O-C₂₋₆ alkenyl,-Thr(Pr1)-Arg(Pr2)-Arg(Pr3)-O-tri-C₁₋₃ alkylsilyl, -Thr(Pr1)-OH, -Thr(Pr1)-NH₂, -Thr(Pr1)-O-C₁₋₆ alkyl, -Thr(Pr1)-O-C₂₋₆ alkenyl and -Thr(Pr1)-O-tri-C₁₋₃ alkylsilyl, a peptide residue comprising 1 to 200 amino acids comprising optionally protected side chain and optionally protected terminal carboxyl group;
wherein Pr1 is hydrogen or a -OH protecting group and each Pr2 and PR3 is independently hydrogen or a guanidine protecting group;
P is hydrogen, an amino protecting group, an N-terminus peptide residue comprising 1 to 200 amino acids comprising optionally protected side chain and optionally protected terminal amino group, wherein the N-terminus peptide residue comprises a C-terminus and an N-terminus;
provided that when R is selected from the group consisting of -OH, a carboxyl protecting group, -NH₂, -O-C₁₋₆ alkyl, -O-C₂₋₆ alkenyl, -O-tri-C₁₋₃ alkyl silyl, a peptide residue selected from the group consisting of -Pro-OH, -Pro-NH₂, -Pro-O-C₁₋₆ alkyl, -Pro-O-C₂₋₆ alkenyl, -Pro-O-tri-C₁₋₃ alkylsilyl, Thr(Pr1), -Thr(Pr1)-Arg(Pr2)-Arg(Pr3)-OH,-Thr(Pr1)-Arg(Pr2)-Arg(Pr3)-NH₂, -Thr(Pr1)-Arg(Pr2)-Arg(Pr3)-O-C1-6 alkyl, -Thr(Pr1)-Arg(Pr2)-Arg(Pr3)-O-C₂₋₆ alkenyl, -Thr(Pr1)-Arg(Pr2)-Arg(Pr3)-O-tri-C₁₋₃ alkylsilyl,-Thr(Pr1)-OH, -Thr(Pr1)-NH₂, -Thr(Pr1)-O-C₁₋₆ alkyl, Thr(Pr1)-O-C₂₋₆ alkenyl and-Thr(Pr1)-O-tri-C₁₋₃ alkylsilyl, then P is not hydrogen or an amino protecting group;
coupling the lysine-resin conjugate of the formula I, wherein R is selected from - OH, a carboxyl protecting group and a peptide residue comprising 1 to 200 amino acids comprising optionally protected side chain and optionally protected terminal carboxyl group, with a protected, partially protected or unprotected peptide residue Ib, where the peptide residue comprising 1 to 200 amino acids and the peptide residue Ib together comprises the insulin B-chain or the insulin B-chain derivative, to form the protected, partially protected or unprotected insulin B-chain or an insulin B-chain derivative.

2. The method of Claim 1, wherein the resin is a 2-chlorotrityl resin.

3. The method of any one of Claims 1 or 2, wherein P is selected from the group consisting of tert-butyloxycarbonyl (Boc), 9-fluorenylmethyloxycarbonyl (Fmoc), benzyloxy-carbonyl (carboxybenzyl or Z), 1-(4,4-dimethyl-2,6-dioxocyclohex-1-ylidene)-ethyl (Dde), 2-nitrophenylsulfenyl (Nps) and allyloxycarbonyl (alloc).

4. The method of any one of Claims 1-3, wherein the partially protected or unprotected insulin B-chain or an insulin B-chain derivative is selected from the group consisting of the B chain of insulin glargin (SEQ ID NO: 23), the B chain of SEQ ID NO: 5 (insulin Lispro), the B chain of insulin detemir (SEQ ID NO: 27), the B chain of SEQ ID NO: 7 (insulin aspart), the B chain of insulin degludec (SEQ ID NO: 29), and analogs or derivatives thereof; and
further cleaving of the resin-bound insulin B-chain or an insulin B-chain derivative by contacting the resin-bound peptide under mild acidic condition using a mixture of an organic acid and an alcoholic solvent, or by heating the resin-bound peptide to an elevated temperature, or both using a mixture of an organic acid and an alcoholic solvent along with heating the resin-bound peptide at an elevated temperature for a sufficient period of time to cleave the insulin B-chain or an insulin B-chain derivative from the resin.

5. The method of Claim 4, wherein the organic acid is selected from the group consisting of trifluoroacetic acid and acetic acid, and mixtures thereof, the alcoholic solvent is selected from the group consisting of trifluoroethanol, hexafluoro-isopropanol, methanol and mixtures thereof, and heating of the resin bound peptide is performed with microwaves.

6. The method of Claim 4, wherein the partially protected or unprotected insulin B-chain or an insulin B-chain derivative is selected from the group consisting of an N-acylated derivative, a pegylated derivative, a biotinylated derivative, a derivative comprising a chromophore and a peptide residue comprising a natural amino acid residue, an unnatural amino acid residue, and mixtures thereof.

7. A method for preparing an insulin B-chain peptide selected from SEQ ID NOs: 3, 23, 27, 29 and 34 comprising cleaving the peptide from a peptide-resin conjugate of the formula IV:
W-AA₁-AAₘ IV
wherein:
W is a resin of a formulae IIIa, IIIb, IIIc or IIId: 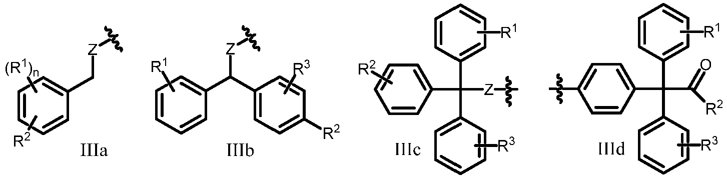 wherein:
n is 0, 1, 2 or 3;
each R¹ and R³ is independently selected from H or is independently selected from the group consisting of 2-Cl, 2-C₁₋₃ alkyl, 2-C₁₋₃ alkoxy, 4-C₁₋₃ alkyl, 4-C₁₋₃ alkoxy, with the proviso that in each of the formulae IIIb, IIIc and IIId, only one of R¹ and R³ is 2-Cl and only one of R¹ and R³ is H;
R² is the solid phase of the resin; and Z is a bond or -C(=O)-;
AA₁ is a first peptide residue comprising a lysine residue or derivative thereof attached to W by the amino side chain of the lysine or lysine derivative;
and
AAₘ is the second to m number of residues where m is an integer from 1-200; by contacting the peptide-resin conjugate with a mixture of an organic acid and a solvent, or by heating the peptide-resin to an elevated temperature, or both using a mixture of an organic acid and a solvent, along with heating the resin-bound peptide at an elevated temperature for a sufficient period of time to cleave the peptide residue from the resin W.

8. The method of Claim 7, wherein the peptide-resin conjugate is selected from the group consisting of SEQ ID NOs: 17, 22 and 24.

9. The method of Claim 8, wherein the peptide-resin conjugate selected from SEQ ID NO: 17 is further deprotected to form human B-chain of SEQ ID NO: 3.

10. The method of any one of Claims 7-9 wherein the organic acid is selected from the group consisting of trifluoroacetic acid and acetic acid, and mixtures thereof, the alcoholic solvent is selected from the group consisting of trifluoroethanol, hexafluoro-isopropanol, methanol and mixtures thereof, and heating of the peptide-resin conjugate is performed at about 30 to 50 ºC.

## Patentansprüche

1. Verfahren zur Festphasensynthese einer geschützten, teilweise geschützten oder ungeschützten Insulin-B-Kette oder eines Insulin-B-Kette-Derivats, wobei das Verfahren Folgendes umfasst:
das Herstellen eines Lysin-Harz-Konjugats, welches ein Harz und ein Lysin oder ein Lysinderivat der Formel I umfasst, wobei: 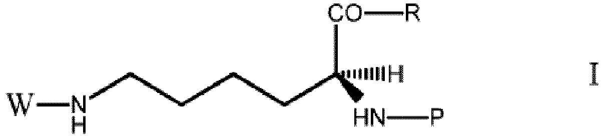 W für einen Rest der Formel IIIa, IIIb, IIIc oder IIId steht: 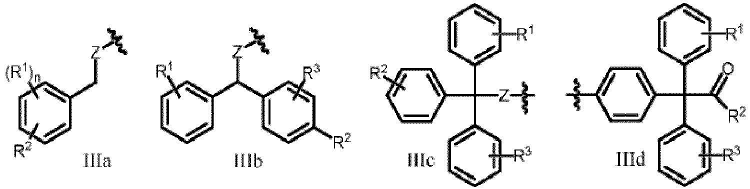 wobei:
n für 0, 1, 2 oder 3 steht,
R¹ und R³ jeweils unabhängig aus H ausgewählt sind oder jeweils unabhängig aus der aus 2-Cl, 2-C₁₋₃-Alkyl, 2-C₁₋₃-Alkoxy, 4-C₁₋₃-Alkyl, 4-C₁₋₃-Alkoxy bestehenden Gruppe ausgewählt sind, mit der Maßgabe, dass in den Formeln IIIb, IIIc und IIId jeweils nur einer der Reste R¹ und R² für 2-Cl steht und nur einer der Reste R¹ und R³ für H steht,
R² für die Festphase des Harzes steht und Z für eine Bindung oder -C(=O)- steht,
R aus der aus -OH, einer Carboxylschutzgruppe, -NH₂, -O-C₁₋₆-Alkyl, -O-C₂₋₆-Alkenyl, -O-Tri-C₁₋₃-alkylsilyl, einem Peptidrest ausgewählt aus der Gruppe bestehend aus -Pro-OH, -Pro-NH₂, -Pro-O-C₁₋₆-Alkyl, -Pro-O-C₂₋₆-Alkenyl, -Pro-O-Tri-C₁₋₃-alkylsilyl, Thr(Pr1), -Thr(Pr1)-Arg(Pr2)-Arg(Pr3)-OH, -Thr(Pr1)-Arg(Pr2)-Arg(Pr3)-NH₂, -Thr(Pr1)-Arg(Pr2)-Arg(Pr3)-O-C1-6-Alkyl, -Thr(Pr1)-Arg(Pr2)-Arg(Pr3)-O-C₂₋₆-Alkenyl, -Thr(Pr1)-Arg(Pr2)-Arg(Pr3)-O-Tri-C₁₋₃-alkylsilyl, -Thr(Pr1)-OH, -Thr(Pr1)-NH₂, -Thr(Pr1)-O-C₁₋₆-Alkyl, -Thr (Pr1) -O-C₂₋₆-Alkenyl und -Thr(Pr1)-O-Tri-C₁₋₃-alkylsilyl, einem Peptidrest mit 1 bis 200 Aminosäuren mit gegebenenfalls geschützter Seitenkette und gegebenenfalls geschützter terminaler Carboxylgruppe bestehenden Gruppe ausgewählt ist,
wobei Pr1 für Wasserstoff oder eine -OH-Schutzgruppe steht und Pr2 und Pr3 jeweils unabhängig voneinander für Wasserstoff oder eine Guanidinschutzgruppe stehen,
P für Wasserstoff, eine Aminoschutzgruppe, einen N-terminalen Peptidrest mit 1 bis 200 Aminosäuren mit gegebenenfalls geschützter Seitenkette und gegebenenfalls geschützter terminaler Aminogruppe steht, wobei der N-terminale Peptidrest einen C-Terminus und einen N-Terminus umfasst,
mit der Maßgabe, dass, wenn R aus der aus -OH, einer Carboxylschutzgruppe, -NH₂, -O-C₁₋₆-Alkyl, -O-C₂₋₆-Alkenyl, -O-Tri-C₁₋₃-alkylsilyl, einem Peptidrest ausgewählt aus der Gruppe bestehend aus -Pro-OH, -Pro-NH₂, -Pro-O-C₁₋₆-Alkyl, -Pro-O-C₂₋₆-Alkenyl, -Pro-O-Tri-C₁₋₃-alkylsilyl, Thr(Pr1), -Thr(Pr1)-Arg(Pr2)-Arg(Pr3)-OH, -Thr(Pra1)-Arg(Pr2)-Arg(Pr3)-NH₂, -Thr(Pr1)-Arg(Pr2)-Arg(Pr3)-O-C1-6-Alkyl, -Thr(Pr1)-Arg(Pr2)-Arg(Pr3)-O-C₂₋₆-Alkenyl, -Thr(Pr1)-Arg(Pr2)-Arg(Pr3)-O-Tri-C₁₋₃-alkylsilyl, -Thr(Pr1)-OH, -Thr(Pr1)-NH₂, -Thr(Pr1)-O-C₁₋₆-Alkyl, -Thr(Pr1)-O-C₂₋₆-Alkenyl und -Thr(Pr1)-O-Tri-C₁₋₃-alkylsilyl bestehenden Gruppe ausgewählt ist, P nicht für Wasserstoff oder eine Aminoschutzgruppe steht,
das Kuppeln des Lysin-Harz-Konjugats der Formel I, wobei R aus -OH, einer Carboxylschutzgruppe und einem Peptidrest mit 1 bis 200 Aminosäuren mit gegebenenfalls geschützter Seitenkette und gegebenenfalls geschützter terminaler Carboxylgruppe ausgewählt ist, mit einem geschützten, teilweise geschützten oder ungeschützten Peptidrest Ib, wobei der Peptidrest mit 1 bis 200 Aminosäuren und der Peptidrest Ib zusammen die Insulin-B-Kette oder das Insulin-B-Kette-Derivat umfassen, unter Bildung der geschützten, teilweise geschützten oder ungeschützten Insulin-B-Kette oder eines Insulin-B-Kette-Derivats.

2. Verfahren nach Anspruch 1, wobei es sich bei dem Harz um ein 2-Chlortritylharz handelt.

3. Verfahren nach einem der Ansprüche 1 oder 2, wobei P aus der aus tert.-Butyloxycarbonyl (Boc), 9-Fluorenylmethyloxycarbonyl (Fmoc), Benzyloxycarbonyl (Carboxybenzyl oder Z), 1-(4,4-Dimethyl-2,6-Dioxocyclohex-1-yliden)ethyl (Dde), 2-Nitrophenylsulfenyl (Nps) und Allyloxycarbonyl (alloc) bestehenden Gruppe ausgewählt ist.

4. Verfahren nach einem der Ansprüche 1-3, wobei die teilweise geschützte oder ungeschützte Insulin-B-Kette oder ein Insulin-B-Kette-Derivat aus der aus der B-Kette von Insulin glargin (SEQ ID NO: 23), der B-Kette von SEQ ID NO: 5 (Insulin Lispro), der B-Kette von Insulin detemir (SEQ ID NO: 27), der B-Kette von SEQ ID NO: 7 (Insulin aspart), der B-Kette von Insulin degludec (SEQ ID NO: 29) und Analoga oder Derivaten davon bestehenden Gruppe ausgewählt ist und
bei dem man weiterhin die harzgebundene Insulin-B-Kette oder ein Insulin-B-Kette-Derivat abspaltet, indem man das harzgebundene Peptid unter Anwendung einer Mischung einer organischen Säure und eines alkoholischen Lösungsmittels milden sauren Bedingungen aussetzt oder indem man das harzgebundene Peptid auf eine erhöhte Temperatur erhitzt oder indem man sowohl eine Mischung einer organischen Säure und eines alkoholischen Lösungsmittels verwendet als auch das harzgebundene Peptid über eine zum Abspalten der Insulin-B-Kette bzw. eines Insulin-B-Kette-Derivats vom Harz ausreichende Zeitspanne auf eine erhöhte Temperatur erhitzt.

5. Verfahren nach Anspruch 4, wobei die organische Säure aus der aus Trifluoressigsäure und Essigsäure und Mischungen davon bestehenden Gruppe ausgewählt ist, das alkoholische Lösungsmittel aus der aus Trifluorethanol, Hexafluorisopropanol, Methanol und Mischungen davon bestehenden Gruppe ausgewählt ist und das Erhitzen des harzgebundenen Peptids mit Mikrowellen erfolgt.

6. Verfahren nach Anspruch 4, wobei die teilweise geschützte oder ungeschützte Insulin-B-Kette oder ein Insulin-B-Kette-Derivat aus der aus einem N-acylierten Derivat, einem pegylierten Derivat, einem biotinylierten Derivat, einem ein Chromophor und einen Peptidrest mit einem natürlichen Aminosäurerest, einem unnatürlichen Aminosäurerest und Mischungen davon umfassenden Derivat ausgewählt ist.

7. Verfahren zur Herstellung eines Insulin-B-Kette-Peptids ausgewählt aus SEQ ID NOs: 3, 23, 27, 29 und 34, bei dem man das Peptid von einem Peptid-Harz-Konjugat der Formel IV:
W-AA₁-AAₘ IV
abspaltet,
wobei:
W für einen Rest der Formel IIIa, IIIb, IIIc oder IIId steht: 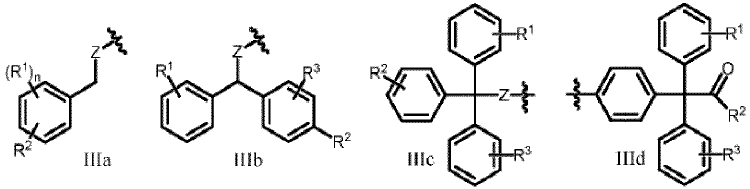 wobei:
n für 0, 1, 2 oder 3 steht,
R¹ und R³ jeweils unabhängig aus H ausgewählt sind oder jeweils unabhängig aus der aus 2-Cl, 2-C₁₋₃-Alkyl, 2-C₁₋₃-Alkoxy, 4-C₁₋₃-Alkyl, 4-C₁₋₃-Alkoxy bestehenden Gruppe ausgewählt sind, mit der Maßgabe, dass in den Formeln IIIb, IIIc und IIId jeweils nur einer der Reste R¹ und R² für 2-Cl steht und nur einer der Reste R¹ und R³ für H steht,
R² für die Festphase des Harzes steht und Z für eine Bindung oder -C(=O)- steht,
AA₁ für einen ersten Peptidrest steht, welcher einen Lysinrest oder ein Derivat davon gebunden an W über die Aminoseitenkette des Lysins bzw. Lysinderivats umfasst,
und
AAₘ für den zweiten bis m-tem Rest steht, wobei m für eine ganze Zahl von 1-200 steht,
durch Inkontaktbringen des Peptid-Harz-Konjugats mit einer Mischung einer organischen Säure und eines Lösungsmittels oder durch Erhitzen des Peptid-Harzes auf eine erhöhte Temperatur oder indem man sowohl eine Mischung einer organischen Säure und eines Lösungsmittels anwendet und das harzgebundene Peptid über einem zum Abspalten des Peptidrestes von dem Harz W ausreichende Zeitspanne auf eine erhöhte Temperatur erhitzt.

8. Verfahren nach Anspruch 7, wobei das Peptid-Harz-Konjugat aus der aus SEQ ID NOs: 17, 22 und 24 bestehenden Gruppe ausgewählt ist.

9. Verfahren nach Anspruch 8, wobei das Peptid-Harz-Konjugat, das aus SEQ ID NO: 17 ausgewählt ist, weiter unter Bildung der humanen B-Kette der SEQ ID NO: 3 entschützt wird.

10. Verfahren nach einem der Ansprüche 7-9, wobei die organische Säure aus der aus Trifluoressigsäure und Essigsäure und Mischungen davon bestehenden Gruppe ausgewählt ist, das alkoholische Lösungsmittel aus der aus Trifluorethanol, Hexafluorisopropanol, Methanol und Mischungen davon bestehenden Gruppe ausgewählt ist und das Erhitzen des Peptid-Harz-Konjugates bei etwa 30 bis 50°C erfolgt.

## Revendications

1. Procédé de synthèse en phase solide d'une chaîne B d'insuline protégée, partiellement protégée ou non protégée ou un dérivé de chaîne B d'insuline, le procédé comprenant :
la préparation d'un conjugué lysine-résine comprenant une résine et une lysine ou un dérivé de lysine de formule I, dans lequel : 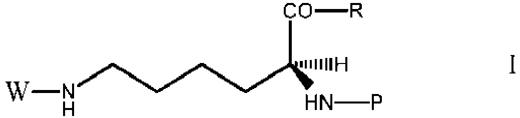 W est une résine de formule IIIa, IIIb, IIIc ou IIId : 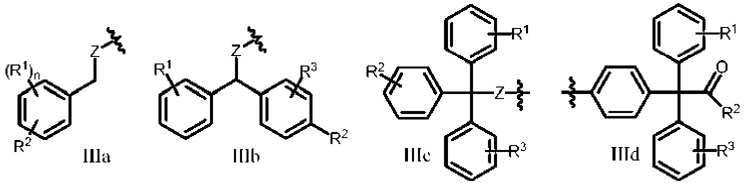 dans laquelle :
n est 0, 1, 2 ou 3 ;
chaque R¹ et R³ est indépendamment choisi parmi H ou est indépendamment choisi dans le groupe constitué de 2-C1, 2- (alkyle en C₁₋₃), 2- (alcoxy en C₁₋₃), 4- (alkyle en C₁₋₃), 4-(alcoxy en C₁₋₃), à condition que, dans chacune des formules IIIb, IIIc et IIId, un seul de R¹ et R³ est 2-Cl et un seul de R¹ et R³ est H ;
R² est la phase solide de la résine ; et Z est une liaison ou -C(=O)- ;
R est choisi dans le groupe constitué de -OH, un groupe protecteur de carboxyle, -NH₂, -O-(alkyle en C₁₋₆), -O-(alcényle en C₂₋₆), -O-tri-(alkyle en C₁₋₃)silyle, un résidu peptidique choisi dans le groupe constitué de -Pro-OH, -Pro-NH₂, -Pro-O-(alkyle en C₁₋₆), -Pro-O-(alcényle en C₂₋₆), -Pro-O-tri-(alkyle en C₁₋₃)silyle, Thr(Pr1), -Thr(Pr1)-Arg(Pr2)-Arg(Pr3)-OH, -Thr(Pr1)-Arg(Pr2)-Arg(Pr3)-NH₂, -Thr(Pr1)-Arg(Pr2)-Arg(Pr3)-O-(alkyle en C₁₋₆), -Thr(Pr1)-Arg(Pr2)-Arg(Pr3)-O-(alcényle en C₂₋₆), -Thr(Pr1)-Arg(Pr2)-Arg(Pr3)-O-tri-(alkyle en C₁₋₃)silyle, -Thr(Pr1)-OH, -Thr(Pr1)-NH₂, -Thr(Pr1)-O-(alkyle en C₁₋₆), -Thr(Pr1)-O-(alcényle en C₂₋₆) et -Thr(Pr1)-O-tri-(alkyle en C₁₋₃)silyle, un résidu peptidique comprenant 1 à 200 acides aminés comprenant une chaîne latérale facultativement protégée et un groupe carboxyle terminal facultativement protégé ;
où Pr1 est hydrogène ou un groupe protecteur de -OH et chaque Pr2 et PR3 est indépendamment hydrogène ou un groupe protecteur de guanidine ;
P est hydrogène, un groupe protecteur d'amino, un résidu peptidique N-terminal comprenant 1 à 200 acides aminés comprenant une chaîne latérale facultativement protégée et un groupe amino terminal facultativement protégé, le résidu peptidique N-terminal comprenant une extrémité C-terminale et une extrémité N-terminale ;
à condition que lorsque R est choisi dans le groupe constitué de -OH, un groupe protecteur de carboxyle, -NH₂, -O-(alkyle en C₁₋₆), -O-(alcényle en C₂₋₆), -O-tri-(alkyle en C₁₋₃)silyle, un résidu peptidique choisi dans le groupe constitué de -Pro-OH, -Pro-NH₂, -Pro-O-(alkyle en C₁₋₆), -Pro-O-(alcényle en C₂₋₆), -Pro-O-tri-(alkyle en C₁₋₃)silyle, Thr(Pr1), -Thr(Pr1)-Arg(Pr2)-Arg(Pr3)-OH, -Thr(Pr1)-Arg(Pr2)-Arg(Pr3)-NH₂, -Thr(Pr1)-Arg(Pr2)-Arg(Pr3)-O-(alkyle en C₁₋₆), -Thr(Pr1)-Arg(Pr2)-Arg(Pr3)-O-(alcényle en C₂₋₆), -Thr(Pr1)-Arg(Pr2)-Arg(Pr3)-O-tri-(alkyle en C₁₋₃)silyle, -Thr(Pr1)-OH, -Thr(Pr1)-NH₂, -Thr(Pr1)-O-(alkyle en C₁₋₆), -Thr(Pr1)-O-(alcényle en C₂₋₆) et -Thr(Pr1)-O-tri-(alkyle en C₁₋₃)silyle, alors P n'est pas hydrogène ou un groupe protecteur d'amino ;
le couplage du conjugué lysine-résine de formule I, dans lequel R est choisi parmi -OH, un groupe protecteur de carboxyle et un résidu peptidique comprenant 1 à 200 acides aminés comprenant une chaîne latérale facultativement protégée et un groupe carboxyle terminal facultativement protégé, avec un résidu peptidique Ib protégé, partiellement protégé ou non protégé, où le résidu peptidique comprenant 1 à 200 acides aminés et le résidu peptidique Ib comprend la chaîne B d'insuline ou le dérivé de chaîne B d'insuline, pour former la chaîne B d'insuline protégée, partiellement protégée ou non protégée ou un dérivé de chaîne B d'insuline.

2. Procédé de la revendication 1, dans lequel la résine est une résine 2-chlorotrityle.

3. Procédé de l'une quelconque des revendications 1 ou 2, dans lequel P est choisi dans le groupe constitué de tert-butyloxycarbonyle (Boc), 9-fluorényl-méthyloxycarbonyle (Fmoc), benzyloxy-carbonyle (carboxybenzyle ou Z), 1-(4,4-diméthyl-2,6-dioxocyclohex-1-ylidène)-éthyle (Dde), 2-nitrophényl-sulfényle (Nps) et allyloxycarbonyle (alloc).

4. Procédé de l'une quelconque des revendications 1 à 3, dans lequel la chaîne B d'insuline partiellement protégée ou non protégée ou un dérivé de chaîne B d'insuline est choisi dans le groupe constitué de la chaîne B de l'insuline glargine (SEQ ID NO: 23), la chaîne B de SEQ ID NO: 5 (insuline lispro), la chaîne B de l'insuline détémir (SEQ ID NO: 27), la chaîne B de SEQ ID NO: 7 (insuline asparte), la chaîne B de l'insuline dégludec (SEQ ID NO: 29), et des analogues ou dérivés de ceux-ci ; et
comprenant en outre le clivage de la chaîne B d'insuline ou d'un dérivé de chaîne B lié à la résine par mise en contact du peptide lié à la résine dans des conditions légèrement acides au moyen d'un mélange d'un acide organique et d'un solvant alcoolique, ou par chauffage du peptide lié à la résine à une température élevée, ou à la fois en utilisant un mélange d'un acide organique et d'un solvant alcoolique avec chauffage du peptide lié à la résine à une température élevée pendant un temps suffisant pour cliver la chaîne B d'insuline ou un dérivé de chaîne B d'insuline depuis la résine.

5. Procédé de la revendication 4, dans lequel l'acide organique est choisi dans le groupe constitué de l'acide trifluoroacétique et l'acide acétique, et des mélanges de ceux-ci, le solvant alcoolique est choisi dans le groupe constitué du trifluoroéthanol, de l'hexafluoro-isopropanol, du méthanol et de mélanges de ceux-ci, et le chauffage du peptide lié à la résine est effectué avec des micro-ondes.

6. Procédé de la revendication 4, dans lequel la chaîne B d'insuline partiellement protégée ou non protégée ou un dérivé de chaîne B d'insuline est choisi dans le groupe constitué d'un dérivé N-acylé, un dérivé pégylé, un dérivé biotinylé, un dérivé comprenant un chromophore et un résidu peptidique comprenant un résidu d'acide aminé naturel, un résidu d'acide aminé non naturel, et des mélanges de ceux-ci.

7. Procédé de préparation d'un peptide de chaîne B d'insuline choisi parmi SEQ ID NO: 3, 23, 27, 29 et 34 comprenant le clivage du peptide à partir d'un conjugué peptide-résine de formule IV :
W-AA₁-AAₘ IV
dans laquelle :
W est une résine de formule IIIa, IIIb, IIIc ou IIId : 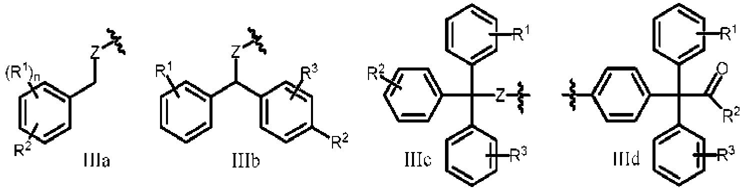 dans laquelle :
n est 0, 1, 2 ou 3 ;
chaque R¹ et R³ est indépendamment choisi parmi H ou est indépendamment choisi dans le groupe constitué de 2-Cl, 2- (alkyle en C₁₋₃), 2- (alcoxy en C₁₋₃), 4- (alkyle en C₁₋₃), 4-(alcoxy en C₁₋₃), à condition que, dans chacune des formules IIIb, IIIc et IIId, un seul de R¹ et R³ soit 2-Cl et un seul de R¹ et R³ soit H ;
R² est la phase solide de la résine ; et Z est une liaison ou -C(=O)- ;
AA₁ est un premier résidu peptidique comprenant un résidu lysine ou un dérivé de celui-ci lié à W par la chaîne latérale amino de la lysine ou du dérivé de lysine ; et
AAₘ est le deuxième à m^{ième} numéro de résidus où m est un entier de 1 à 200 ; par mise en contact du conjugué peptide-résine avec un mélange d'un acide organique et d' un solvant, ou par chauffage du peptide-résine à une température élevée, ou en utilisant à la fois un mélange d'un acide organique et d'un solvant, sous chauffage du peptide lié à la résine à une température élevée pendant une durée suffisante pour cliver le résidu peptidique à partir de la résine W.

8. Procédé de la revendication 7, dans lequel le conjugué peptide-résine est choisi dans le groupe constitué de SEQ ID NO: 17, 22 et 24.

9. Procédé de la revendication 8, dans lequel le conjugué peptide-résine est choisi parmi SEQ ID NO: 17 est en outre déprotégé pour former la chaîne B humaine de SEQ ID NO: 3.

10. Procédé de l'une quelconque des revendications 7 à 9 dans lequel l'acide organique est choisi dans le groupe constitué de l'acide trifluoroacétique et l'acide acétique, et des mélanges de ceux-ci, le solvant alcoolique est choisi dans le groupe constitué du trifluoroéthanol, de l'hexafluoro-isopropanol, du méthanol et de mélanges de ceux-ci, et le chauffage du conjugué peptide-résine est effectué à environ 30 à 50 °C.
